# EUROPEAN PATENT SPECIFICATION

(11) **EP 1 292 340 B1**
(45) Date of publication and mention of the grant of the patent: **19.10.2005**
(21) Application number: 01934009.0
(22) Date of filing: 23.05.2001
(51) Int. Cl.: A61L 26/00, A61L 24/00, A61K 48/00

(54) **TRANSFECTION SYSTEM**
TRANSFEKTIONSSYSTEM
SYSTEME DE TRANSFECTION

(30) Priority: 24.05.2000 DE 10025609
(43) Date of publication of application: 19.03.2003
(73) Proprietor: Universitätsklinikum Freiburg, 79104 Freiburg (DE)
(72) Inventor: ANDREE, Christoph, 79244 Umkirch (DE); VOIGT, Matthias, 79102 Freiburg (DE); STARK, G., Björn, 79874 Breitnau (DE)
(74) Representative: Müller-Boré & Partner Patentanwälte
(86) International application number: PCT/EP2001/005937
(87) International publication number: WO 2001/089593

(56) References cited:
- WO-A-97/38729
- DE-A- 19 716 098
- US-A- 5 487 889

## Description

The present invention relates to matrix-mediated transfection systems, wherein the matrix consists of a self-hardening biopolymer. Moreover, the invention relates to a method of producing a preparation containing plasmid DNA, a component of the self-hardening material, and a cell suspension of the cells to be transfected, the preparation itself and its use for treating tissue injuries and changes.

In particular, the present invention relates to a fibrin-mediated transfection system for cells for improved wound healing, tissue regeneration and tissue repair.

### Background of the Invention

In medicine, tissue defects and their treatment represent a great problem. Both in surgical interventions and also as a consequence of wear, of external influences such as injuries caused by burns, a stroke, etc., corresponding traumas of the tissue occur the healing and regeneration of which is decisive. Also in many illnesses tissue is damaged, psoriasis and arthritis being mentioned by way of example.

It is therefore an object to develop methods and possible ways of improving and accelerating these healing and regeneration processes. Particularly when large areas of tissue are damaged, such as skin damage, bone damage, cartilage damage or in persons having a predisposition therefore, whose own capacities of regeneration and healing are restricted, it is necessary to assist this natural process, particularly also from the point of view that this regeneration should be such that it does not differ from its natural surroundings, i.e. does not lead to a restricted or changed tissue, e.g. scar formation, due to unnatural growth. For this purpose, i.a. matrices are used which are intended to assist in the healing process. These matrices often are constructed such that they release factors which positively aid the healing process.

In conventional methods, a transplantation of keratinocytes is, e.g., used for large-area burns. These are applied to the wound as autologous or allogenic grafts. These cells are grafted on the wound as so-called sheets, i.e. as multiple cell layers. For this purpose it is necessary to previously recover keratinocytes from the individual, to isolate then and to culture them in vitro so as to form multiple-layer cell sheets comprising a supportive matrix so that these may then be grafted onto the wound.

A further possible way of accelerating the regeneration of the damaged tissue is the external administration of promoting factors. On the one hand, these factors may promote the growth of the cells involved in the regeneration process. On the other hand, these factors may also inhibit processes which counteract a rapid regeneration. So far, these factors which primarily are growth factors have been supplied to the injured tissue in many different ways, such as, e.g., by external application. However, this has the disadvantage that high doses had to be supplied. Moreover, most of the factors have only a short half-life in vivo so that multiple administrations had been necessary. When administering factors externally, there is also a risk that they might contain contaminants. Both, when purifying material from natural sources and in the recombinant production of the factors there is a risk of the preparations containing contaminants which may negatively affect the progression of regeneration. Therefore, the external administration of mediators, such as growth factors, proved to be an inefficient system, mainly also from the point of view that some mediators cannot at all be administered externally.

Therefore, it has been attempted recently to produce these growth factors in vivo by transfection of cells (gene transfer). Andree et al. (PNAS, 91, pp. 12188-12192, 1994) were able to demonstrate that in vivo transfer of a plasmid which contains a gene coding for a growth factor for the epidermis in keratinocytes improves wound healing in the animal model. Gene transfer was carried out in a conventional method by means of a so-called "gene gun", wherein the plasmids coupled to a carrier are applied to the wound and brought into the cells present therein. Subsequently, the cells are unspecifically transfected with the plasmids. Despite a rapid healing of the wound, it has been observed with this transfection system that the factor is only expressed by cells which were present at the edge of the wound at this point of time. Moreover, a specific transfection is not possible with this method. A systematic administration of factors is, however, desirable. The transfection of cells is particularly suitable for this.

So far, however, substantially only cell transfection methods have been known in which the DNA is introduced into the cell by external carriers. Yet in doing so, also the carrier gets into the cell, which may have negative consequences.

Known transfection methods include the viral transfection with retroviruses, adenoviruses or other viruses. In the transfection of eukaryotic cells, the greatest problem is the effectiveness of the transfection. Especially with viral vectors, their tropism, i.e. their affinity relative to certain cell types, still is a major problem. For this reason, adenoviral vectors are already being used, which have a broad tropism and are also capable of transfecting cells, such as muscle cells, hepatocytes, synovial cells, epithelial cells and the like. Moreover, adenoviruses are capable of transfecting cells which do not divide.

Yet the transfection of cells by viral vectors also has some disadvantages. Since the position where integration of the vector occurs is random, these cells may be transformed, and genes which are critical for the life of the cell may be changed. Viruses and viral vectors may have a high rate of polymorphism, and this may lead to inactive vectors or genes. As has already been mentioned, the tropism of the viral vectors is a further problem.

Adenoviruses, although capable of transfecting non-proliferating cells, also have the afore-mentioned disadvantages of viral vectors, i.e. they integrate randomly into the genome of the target cell. Thereby other important genes may be destroyed. The polymorphism rates in viruses are high and, last but not least, these recombinantly prepared viruses have to be propagated and processed for transfection.

Other transfection systems with non-viral compositions are, e.g., liposome-mediated method (e.g. Lipofektin®). A disadvantage of these liposomes is, e.g., a remaining cytotoxicity of the liposomal transfection agent concerning the target cells. This means that the cells to be transfected (target cells) are damaged by the transfection agent itself, causing them to die.

Furthermore, polycationic systems, systems using calcium (calcium-phosphate-DNA precipitations), DEAE-dextrane transfections and others have been described.

As mechanical methods for the transfection of cells, electroporation of cells or the afore-mentioned gene gun method are known.

With the gene gun method, the nude DNA or the DNA associated with liposomes or other carrier molecules can directly be shot onto the cells to be transfected. Although this method partially allows for the transfection of cells without a carrier, it is, however, a random transfection of the cell without allowing to determine the amount of material transfected. Neither is there a specificity of the system, primarily in the transient transfection of cells. The transfection by means of electroporation has the disadvantage that the transfection is performed in vitro in a special apparatus. At first, the cells have to be propagated. This requires various method steps, and the risk of a contamination of the cell suspension is high. There have already been attempts to transfect DNA directly into cells without the use of auxiliary agents. Yet with this approach, the transfection efficiency is too low.

Therefore, it is necessary to develop other transfection systems which overcome the above-mentioned disadvantages.

In WO 97/38729 it has been described that an improved transfection efficiency of cells can be attained if the patient to be treated receives the plasmid together with a matrix. Cells which migrate into the damaged region where this matrix has been applied together with: the plasmid will then exhibit a more effective transfection. With this method it is necessary that the cells migrate to the matrix so as to take up the plasmid. This also means that there is no homogenous distribution of the cells during the regeneration of the damaged region, but that the cells will be transfected from the edge onwards. Thus, there is no simultaneous transfection of the cells.

In DE 197 16 098 A1 it has been described that fibroblasts transfected with a foreign gene can be used for the treatment of wounds, with the foreign gene encoding a factor promoting wound healing. Yet these fibroblasts merely constitute a medium which is to avoid the external administration of the factor that promotes wound healing.

The administered fibroblasts themselves have been irradiated such that they can no longer divide. Thus, they themselves do not participate as cells in wound healing. Moreover, the fibroblast cell line described in DE 197 16 098 constitutes an immortalized cell line which is permanently transfected with the plasmid. A permanent transfection of the cells, however, is not always desirable, instead a transient transfection of the cells is preferred so that expression of the factor encoded by the plasmid will occur only for a certain period of time. When wound healing has been finished, continued expression of this factor is not desirable. On the contrary, this could, e.g., promote scar formation and an abnormal formation of the tissue structure which is unwanted.

Recently, Horch et al. (Cell Transplantation, 7, 3, pp. 309-317, 1998) have described that in the nude mouse animal model, a single-cell suspension of cultured keratinocytes has been applied to the wound with the assistance of a fibrin adhesive. In this manner it has been possible to reconstruct an epidermis of good quality. With this method it has already become possible to shorten the time period between removal of the cells and transplanting of the expanded cells.

Thus, it has been an object of the present invention to provide a simple, immediately available system with which, with minimal time requirements and with as little surgical intervention as possible, in patients suffering from major - acute or chronic - tissue injuries, cells, in particular autologous cells, are provided which are transfected with genes that code for substances exerting a positive effect on wound healing.

### Summary of the Invention

The present invention relates to a method for preparing a composition for wound healing, and for repairing and regenerating tissue. According to the invention, this is achieved by the following steps:
a. providing a plasmid DNA which encodes a gene that has a positive effect on the progression of the regeneration of the tissue, substantially in pure form,
b. providing the component/components of a self-hardening biopolymer, and
c. providing a cell suspension with cells which promote regeneration,
characterized in that components (a), (b) and (c) are incubated with each other simultaneously or successively so that the plasmid and the cell suspension are obtained homogenously distributed in one of the biopolymer components.

A further aspect of the present invention relates to a composition for wound healing, and for the repair and regeneration of the damaged tissue itself. This composition comprises
a. a plasmid DNA which encodes a gene that has a positive effect on the course of wound healing and on the repair and regeneration of the tissue, in substantially pure form;
b. component(s) of a self-hardening biopolymer, and
c. a cell suspension of cells involved in wound healing and in the repair and regeneration of the damaged tissue.

In a further aspect of the invention, this preparation can be used for the treatment of damaged tissue and for the treatment of wounds. Moreover, the invention relates to a kit containing a plasmid which comprises a gene coding for a substance, preferably for a protein which has a positive effect on the wound healing process and on the regeneration process of the damaged tissue, a cell suspension of cells involved in the regeneration of the damaged tissue, and a component of a self-hardening biopolymer, as well as optionally the second component of this biopolymer which is required for or facilitates the hardening or solidification.

Moreover, the present invention relates to the manufacture of a medicament for treating tissue defects using a composition prepared according to the present invention, a pharmaceutical composition as defined therein or a kit according to the present invention.

### Description of the Figures

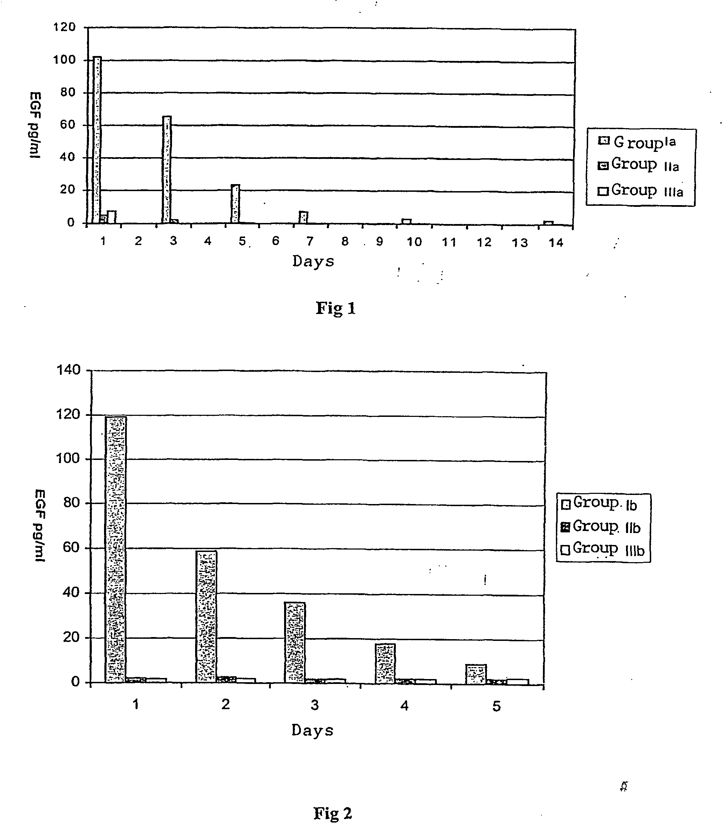

- Fig. 1:: In vitro transfections of cultured human keratinocytes with the EGF-plasmid by using the transfection system according to the invention. The cells were incubated for 3 h with the human EGF-plasmid in the thrombin component (group 1a) or with EGF-plasmid without thrombin (group 2a) or with cells without plasmid and without thrombin (group 3a). EGF protein levels were measured at days 1, 3, 5, 7 10 and 14.
- Fig. 2:: In vitro transfection of non-cultured human keratinocytes with the EGF-plasmid using the transfection system according to the invention. The cells were incubated for 3 h with human EGF-plasmid in the thrombin component (group 1b) or with EGF-plasmid without thrombin (group 2b) or with cells without plasmid and without thrombin (group 3b). EGF protein levels were measured at days 1, 2, 3, 4 and 5.

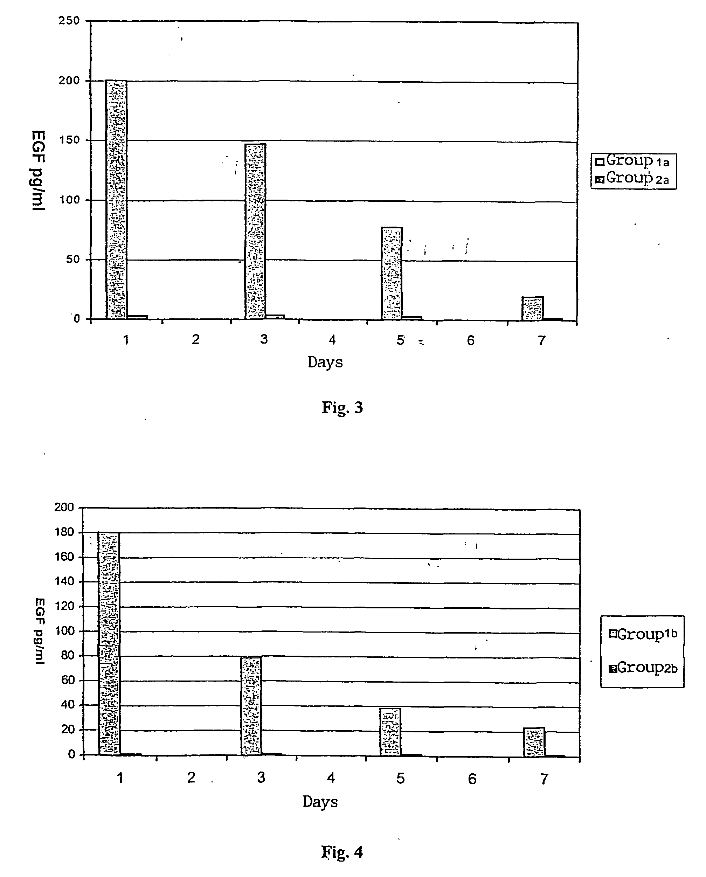

- Fig. 3:: In vivo transfection of cultured human keratinocytes with hEGF-plasmid using the transfection system according to the invention. In group 1a, cells were incubated with EGF, subsequently suspended in the thrombin component of the fibrin adhesive, and transplanted on a full thickness wound of nude mice.
Biopsies were performed at days 1, 3, 5 and 7, and the protein levels in the homogenized wounds were determined by means of ELISA. Control wounds (group 2a) were treated with cells which had been pre-incubated with human b-galactosidase plasmid.
- Fig. 4:: In vivo transfection of non-cultured human keratinocytes with human EGF-plasmid. In group 1b cells were incubated with EGF, subsequently suspended in the thrombin component of the fibrin adhesive and transplanted on a full thickness wound of nude mice.
Biopsies were taken at days 1, 3, 5 and 7, and the protein levels in the homogenized wounds were determined by means of ELISA. Control wounds (group 2b) were treated with cells which had been pre-incubated with human b-galactosidase plasmid.

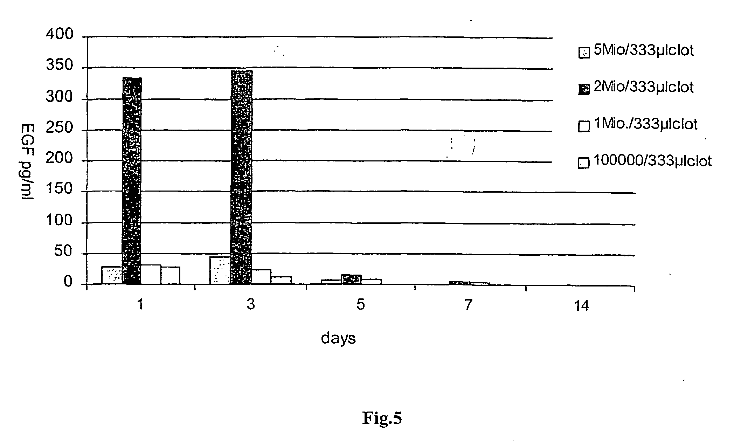

- Fig. 5:: Determination on the optimal keratinocyte concentration. Different numbers of keratinocytes were transfected with 200 µg of EGF-plasmid in 5.7 µl PBS buffer and pre-incubated for 3 hours. The clot volume was 333 µl, EGF expression was measured after 1, 3, 5 and 7 days.

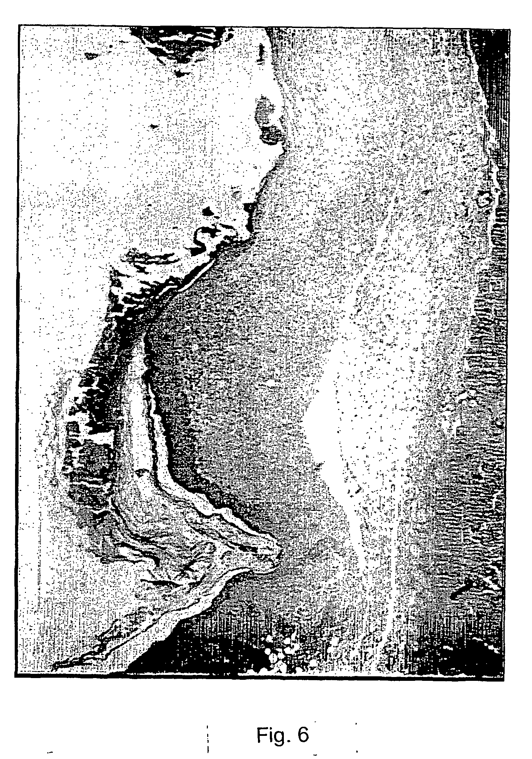

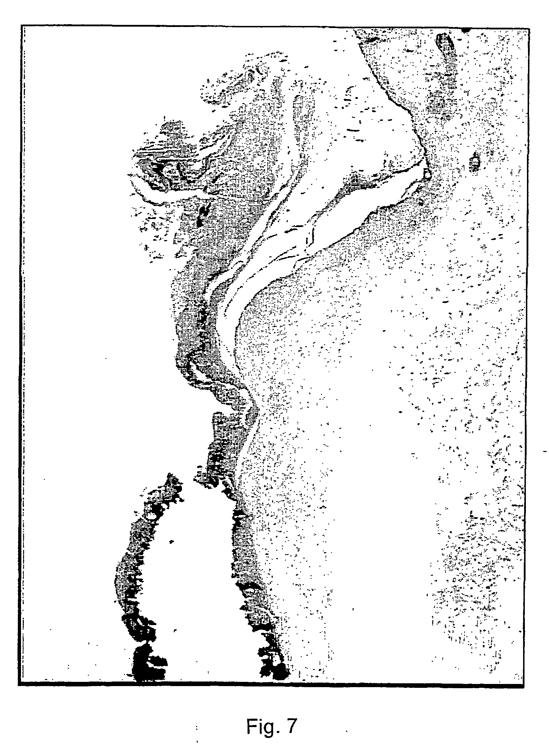

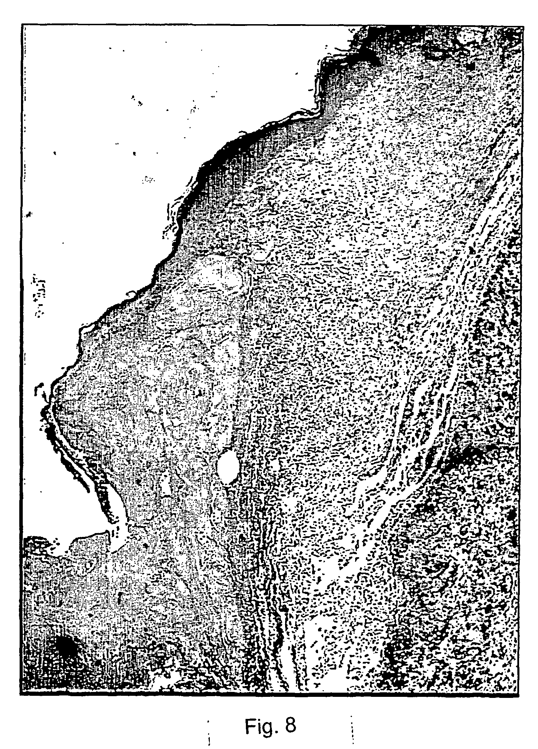

- Figures 6-9:: Histologies of the re-epithelialization of full thickness wounds as described in Example 1 were made after different treatments (groups 1 - 4). For all these investigations the amount of fibrin used was 333 µl, the amount of EGF-plasmid was 200 µg and biopsies were taken on day 12. Magnifications used were 2,5.
- Fig. 6:: Treatment of full thickness wounds with fibrin and EGF-plasmid (group 1).
- Fig. 7:: Treatment of full thickness wounds with fibrin and keratinocytes (group 2).
- Fig. 8:: Treatment of full thickness wounds with fibrin, EGF-plasmid and endothelial cells (group 3).
- Fig. 9:: Treatment of full thickness wounds with fibrin, EGF-plasmid and keratinocytes (group 4).
- Fig.: 10: As Fig. 9 but with a magnification of 10.
- Fig. 11:: Transfection of various cell types with the transfection system according to the invention.

Definitions of terms used in the present invention:
"Tissue defects" herein include both full thickness wounds such as those which occur, e.g., in large-area burns, and also other skin injuries, as well as damage of bones, cartilage, nerves and other tissues.
The "self-hardening biopolymer" is a biologically degradable, biocompatible biopolymer which according to the invention hardens or solidifies by itself by the addition or the presence of a second component. It comprises both natural compositions and synthetic substances.
A "gene with a positive effect" on the regeneration of the damaged or defective tissue includes all DNA sequences which have a promoting effect on regeneration when translated as a protein or polypeptide, but also as an antisense molecule or as a ribozyme.
"Cells involved in wound healing" include both cells which themselves newly form tissue, and cells capable of inhibiting negative factors.
"Repair cells" are cells which are actively involved in the appropriate wound healing and/or repair processes and which can be stimulated and/or activated in using the transfection system according to the present invention.
"Support cells" are not cells which are directly or actively involved in the appropriate wound healing and/or repair processes.

In skin repair for example, for accelerating the process of re-epithelialization, an appropriate repair/support cell pair would be keratinocytes/endothelial cells. Appropriate other pairs of repair/support cells are to be defined according to the wound/tissue to be healed, repaired and/or regenerated.

### Detailed Description of the Invention

The present invention provides a new method which does not require an extra component for the transfection of the cells in vitro, i.e. no auxiliary means, such as viral vectors, liposomes etc., or methods, such as, e.g., electroporation. Furthermore, no transfection-mediating substances are required, such as, e.g. poly-lysine. The method according to the invention for the transfection of cells by means of plasmids is effected by a self-hardening biopolymer matrix. This transfection may be performed both in vitro and in vivo, preferably, however, the transfection is performed ex vivo. What is decisive is that a composition comprising the above-mentioned components, i.e. the cells, the plasmid and the self-hardening biopolymer matrix or a component thereof, respectively, together is applied on or to the damaged area. In doing so, the cells have either been transfected prior to grafting the composition on the damaged region, or the transfection occurs immediately after administration of the composition to the damaged tissue, preferably transfection of the cell occurs ex vivo so that the uniformly distributed cells are present in a transfected state. The self-hardening biopolymer may already be hardened or solidified or gelled when administered to the damaged tissue area or to the tissue defect. Yet hardening may equally occur after administration of the composition to the area to be treated. The composition may, however, also be present in two components, one component containing the self-hardening biopolymer, the cells and the plasmid, whereas the second component contains a further compound for hardening of the biopolymer. The second component may, however, also be provided by the treatment-requiring tissue itself, so to speak in vivo, such as, e.g., thrombin in a fibrin adhesive which then will allow for the hardening of the biopolymer in vivo. The latter possibility is a further preferred form.

Moreover, the present invention provides a transient transfection system comprising a plasmid DNA which codes for a gene that has a positive effect on the progression of regeneration of the tissue, a suspension of cells which are to be transfected, and a self-hardening biopolymer. In particular, the invention relates to a transfection system comprising cells involved in wound healing and in the regeneration and repair of tissue defects, in particular of the skin, with a plasmid encoding a gene which codes for a substance to be expressed, and a self-hardening biopolymer, such as a component/components of a fibrin adhesive

The component of the self-hardening biopolymer allows for a better penetration of the plasmid/uptake by the cells. The non-bound plasmid is taken up by the cells more easily, the transfection rate is highly improved without using further transfection-promoting or mediating substances.

With the assistance of this transfection system it is, moreover, possible to homogenously apply to the damaged area transfected cells which accelerate wound healing and the regeneration of the damaged tissue. Thereby it is possible to completely cover the damaged region, such as, e.g., a full thickness wound so that wound healing and the regeneration of the damaged or defective tissue will occur rapidly. In this instance, the substance expressed by the transfected cells accelerates healing.

The transfection system according to the invention also allows for the ex vivo production of live cell constructs, i.e. of transfected cells, without using other transfection-promoting or mediating substances than the biopolymer component.

More precisely, the present invention provides a simple, immediately available system with which, with minimal time requirements and with as little surgical intervention as possible, in patients suffering from major burn wounds or from chronic wounds, autologous cells are provided which are or have been transfected with genes that code for proteins or peptides, respectively, which have a positive effect on wound healing.

The advantage of the present invention resides in the rapid availability of transfected, autologous/allogenic cells which have the potency of repairing cell defects and simultaneously provide the required substances by autosynthesis in optimal form. Moreover, by the homogenous distribution of the transfected cells it is possible to rapidly attain a therapeutic effect. This is particularly true of wound healing of the skin.

By aid of this system, the immediate availability of transfected cells is increased without requiring prior complex steps of laboratory technology and time-consuming culturing and selection methods which, moreover, harbour the risk of contamination and mean a great loss of time.

Furthermore, the present invention comprises a kit containing the components of the transfection system, i.e. a plasmid which comprises a gene/genes coding for a substance, in particular for a protein/peptide having a positive effect on the regeneration of the tissue, target cells which are transfected, as well as the self-hardening polymer or a component thereof, respectively.

The preparation obtained by the method according to the invention may be used for the treatment of damaged or defective tissue, in particular full thickness wounds, e.g. of the skin. It also allows for the treatment of humans and animals suffering from damaged or defective tissue and full thickness wounds, in particular wounds of the skin. Examples of diseases or disorders preferably treated include burn wounds, bone, muscle, nerve or cartilage defects, chronic wounds or tissue augmentations, especially preferred is a method for wound healing in the skin.

The composition obtained according to the invention allows for the rapid therapeutic treatment of injured or defective tissue. Particularly due to the homogenous distribution of the transfected cells it is possible to quickly and homogenously treat full thickness wounds. In particular, also the inaesthetic scar formation with full thickness wounds can be reduced or avoided.

According to a possible alternative, the target cells which are transfected by the transfection system according to the invention are already present in the matrix in transfected form. These cells are homogenously distributed in the matrix so that a homogenous healing may occur. Thus, according to the invention, a matrix is applied to the damaged tissue which contains the ex vivo transfected target cells which in turn contain genes coding for substances that have a positive effect on the regeneration of the defective tissue. According to a further alternative, however, transfection of the cells may also occur after application in the matrix. By the homogenous distribution of the plasmid and of the cells, a homogenous transfection of the cells is possible which then will lead to a homogenous expression of the factor that has a positive effect on the progress of regeneration.

For the transfection of cells several parameters like e.g. buffers, buffer concentration, time period or ratios of buffer to self-hardening polymer component can be optimized for the different cell types. It was especially found that the presence of CaCl₂, especially in the low amounts used, has no effect on the.transfection efficiency.

According to the invention, "self-hardening polymer" particularly is to be understood as a substance which, following the so-called hardening, will have changed physical properties, e.g. a different viscosity.

Thus, the self-hardening polymer after hardening is, e.g., present as a gel, whereas prior to hardening the substance had a different, in particular lower, viscosity.

In particular, the following compounds may be mentioned as the biopolymer which can be self-hardening and biodegradable: fibrin adhesives or components thereof, collagens, gelatin, alginates, hyaluronic acid, polysaccharides, organic polymers (e.g. PEG, PGA, PLA), derivatives thereof and various combinations of these substances, respectively.

Tissue adhesives based on fibrinogen have been known e.g. from AT-B-359 653, AT-B-359 652 and AT-B-369 990. Besides fibrinogen, they contain a factor which converts fibrinogen to fibrin. This may, e.g., be thrombin. By the action of thrombin, the fibrinogen contained will be converted to fibrin. Factor XIII optionally contained in the tissue adhesive will be activated to factor XIIIa. The latter crosslinks the formed fibrin to a high polymer. The thrombin activity required will be added to the fibrinogen in the form of a thrombin and Ca2+ ion-containing solution. Furthermore, the fibrinogen may contain further proteins, such as fibronectin, plasminogen and albumin.

The tissue adhesive may, e.g., be such as the one described in DE 195 21 324 or in EP 0 345 246; particularly preferred is a fibrin adhesive obtainable under the trademark Tissucol® from Baxter, Germany.

Preferably, the plasmid DNA is mixed into the thrombin component of the fibrin adhesive. Likewise, the cells preferably are added into the thrombin component and are mixed there, since the fibrinogen component has a higher viscosity. The plasmid-DNA and the cell suspension may, however, also be mixed into the fibrinogen component. If the plasmid DNA and the cell suspension are mixed into the fibrinogen component, the thrombin may be provided by the treatment-requiring tissue itself, i.e. in vivo.

The biopolymer according to the invention promotes the efficiency of transfection, yet at the same time it is used as culturing matrix for the cells. This means that transfection and culturing occur in vivo in the same matrix, and no further purifying and isolating steps are required, neither are any further transfection-promoting means necessary. In particular, no transfection-mediating substances, such as, e.g., poly-lysine, are required which act as a "mediator" between the matrix and the plasmid. In other words, the plasmid is not present bound to the matrix, but as a free plasmid.

The matrix component may be present in various forms, both in lyophilized and in liquid form, as a dry powder, microparticles or nanoparticles. Furthermore, it is possible for the biopolymer to be present already as a hardened matrix, a gel-like mass, containing the transfected cells and the plasmid in a homogenous distribution.

Plasmid-biopolymer combinations may be applied to the respective tissue either together or successively, in particular they may be sprayed on.

The cells which according to the invention are used in the method of producing the preparation and thus are contained in the composition according to the invention comprise all types of cells which have a positive effect on tissue regeneration. In particular, these are keratinocytes, thrombocytes, osteoblasts, osteoclasts, fibroblasts, epithelial and endothelial cells, muscle cells, adipocytes, myoblasts, Schwann cells, other connective tissue cells, or precursors thereof etc. Particularly preferred are cells which play a substantial role in wound healing of the skin, such as keratinocytes.

Preferably, the cells are autologous or allogenic cells. The cells as they are to be used according to the invention may have been previously cultured or may be isolated freshly, i.e. have not been cultured.

The plasmid containing a gene/genes which code(s) for a substance that has a positive effect on tissue regeneration may be an expression plasmid, such as is conventionally used for the expression of genes in eukaryotic cells.

The gene/genes or the DNA, respectively, contained in the plasmid may code for protein(s) or peptide(s), respectively, which have a positive effect on tissue regeneration. In particular, this gene codes for factors, such as growth factors, cytokines, therapeutic proteins, hormones and peptide fragments of hormones, cytokine inhibitors, peptidic growth and differentiation factors. By way of example the following should be mentioned here: molecules of the TGF-β superfamily, such as the various TGF-β isoforms; keratinocyte, hepatocyte, epidermal growth factor, PDGF, EGF, VEGF; NGF, erythropoietin, TPA, FGF-1 and FGF-2. Furthermore, hormones, such as growth hormone, PTH etc., peptides which have agonistic or antagonistic activities on receptors or on other molecules which play a role in pathological processes because of a changed expression.

These factors may be used individually or in combination.

The plasmid may contain one or several DNA sequences which encode one or several of the above-indicated factors.

According to the invention, however, it may also encode an antisense-RNA or ribozyme-RNA which then inhibit factors that interfere with wound healing.

The preparation according to the invention and the kit according to the invention may particularly be used for therapeutic applications. They are suitable in the treatment of damaged or defective tissue, such as damaged or defective bones, cartilage, muscles, nerves or skin portions, in particular they are suitable for healing skin wounds. Thus, a further aspect of the present invention is the provision of a pharmaceutical composition for treating defective tissues, comprising a self-hardening biopolymer, a plasmid having the above-indicated properties, and target especially repair cells involved in the process of regenerating the defective tissue, in particular healing skin wounds.

These pharmaceutical compositions may be present in several components. Apart from the target cells which are added shortly before the pharmaceutical preparation is being used, the pharmaceutical composition may be provided in liquid, lyophilized or deep-frozen form.

The fields of use of the pharmaceutical composition particularly comprise human medicine, veterinary medicine and dental medicine.

The relative amounts of plasmid, biopolymer component and cells can be precisely adjusted, or varied, respectively, depending on the type of tissue to be treated. For example, the ratio of plasmid to biopolymer component is at least 5 µg/ml and preferably ranges between 5 and 100 µg/ml. Particularly preferred ranges are 5-25 µg/ml and levels around 50 µg/ml.

The ratio of cells to plasmid ranges, e.g., between 25,000 and 2,500,000 cells/µg of plasmid. Preferred ranges are 25,000- 75,000 cells/µg of plasmid, 75,000-100,000 and 250,000-750,000.

Also the ratio of cells to biopolymer component can be adjusted accordingly and optimized for each cell type and/or wound/tissue to be treated. The numbers of cells per ml of biopolymer may vary in a broad range, e.g. between 100,000 to 8 millions cells per ml of biopolymer component(s).

For keratinocytes a preferred range is between 200,000 and 6 millions, especially preferred is an amount around 3 millions cells per ml of a biopolymer component (=6 millions cells per ml of a clot being formed by a 1:1 mixture of 2 components.

In particular, the ratios also depend on whether an in vitro or an in vivo transfection is carried out. In vivo, in particular, means the transfection of the cells added to the matrix ex vivo, which are homogenously distributed in the matrix with the likewise homogenously distributed plasmid DNA. Thus, e.g., the ratio of cells per ml of biopolymer component in an in vivo transfection is higher by a factor 3-10, preferably 4-6, than in the in vitro transfection.

The present invention will now be described in more detail by way of the following examples.

### Example 1

Increase of the transfection rate by the presence of a self-hardening biopolymer

Expression plasmid: In this example, as the expression plasmid, pCMVβ-Gal and CMVhEGF were used which have already been described previously by Andree et al. (supra). The EGF expression plasmid consists of the very early transcription promoter of the CMV and encodes the secretory signal peptide of the human growth factor (hGF) and the mature EGF polypeptide in an in-frame fusion.

Tissucol® of Baxter, Heidelberg, Germany, was used as the fibrin adhesive. This two-component fibrin adhesive consists of heterologous human plasma protein fractions, the fibrinogen and the thrombin components. The fibrinogen component contains fibrinogen, plasma fibronectin, factor VIII, plasminogen, aprotinin and human albumin. The thrombin component consists of thrombin and calcium chloride.

Cell preparations of the cells were obtained from skin samples following plastic surgery. The keratinocytes were separated from the dermis by using 0.3% dispase (Boehringer, Mannheim, Germany) after a 2 h incubation at 40°C. Epidermal cells subsequently were obtained as single cell suspension by using 0.05% trypsin and 0.02% EDTA (GIBCO, Germany) at 37°C for 30 min and re-suspended in serum-free medium. The cells were used either directly or cultured according to standard protocols (Horch et al., supra).

### Group 1

Cultured (group 1a) and non-cultured (group 1b) human keratinocytes were mixed with CMVh EGF-plasmid for 3 h and subsequently re-suspended in the thrombin component. After re-suspension in thrombin, the fibrin adhesive was put into a 24 well plate and 2 ml of serum-free medium were added.

The medium was exchanged every 24 h and shock-frozen at -70°C.

### Group 2

Cultured and non-cultured keratinocytes (group 2a and group 2b, respectively) were re-suspended in 2 ml medium, containing the CMVhEGF-plasmid, and incubated for 3 h. Subsequently, the cells were put into 24 well-plates and the medium was exchanged every 24 hours and shock-frozen at -70°C.

### Group 3

Cultured (3a) and non-cultured (3b) keratinocytes were re-suspended in 2 ml of medium and then put into 24 well plates. The medium was exchanged every 24 hours and appropriately shock-frozen at -70°C.

The plasmid DNA concentration amounted to 20 µg/ml of thrombin. The expressed protein was detected in vitro by aid of a commercially available ELISA kit (Quantikin, R&D Systems, Minneapolis, USA).

The results of the measurement of the EGF protein in the culture supernatant are represented in Fig. 1. It has been shown that in group 1, an up to 100-fold increase of the EGF protein concentration as compared to the control groups (group 2 and group 3) could be attained. The maximum EGF concentration was measured after 24 h (group 1a: 102.14 µg/ml; group 1b: 119.3 µg/ml ; control 5.1 and 2.26 µg/ml, respectively). After 5 days, a decrease to 23.3 and 8.85 µg/ml, respectively, could be observed). The EGF concentration, measured 14 days after incubation, showed a further decrease for group 1a to 2.24 µg/ml at day 14.

### In vivo-test of the transfection rate

6 to 8 week old nude mice were used as test animals.

### Wounds and grafting methods

2x2 cm sized whole skin wounds (full thickness wounds) were produced on the backs of the anesthethised nude mice. These wounds were made as far as to the panniculus carnosus muscle, and the wound corners were sewed so as to retard wound contraction.

The human keratinocyte suspensions, cultured or non-cultured, were re-suspended in the thrombin portion of the fibrin adhesive. The thrombin component contained either the pCMVβ-Gal or the EGF expression plasmid. Seven wounds of each group were either covered with cultured or with non-cultured keratinocytes and the various plasmids (Table 2). The wounds were covered with a semi-permeable adhesive film (Op-site, Smith & Nephew, Largo, FL) which then is attached to the site and covered with an antimicrobial ointment.

A daily check was made to ensure the integrity of the cover. The experimental groups were grafted either with the hEGF-plasmid (group 1a n=7, group 1b n=7) or with the pCMVβ-Gal plasmid (group 2a n=7, group 2b n=7). The expression of the EGF transgene was monitored at days 1, 3, 5, and 7 by measuring the EGF concentration in the wound homogenisate. At days 1, 3, 5 (two mice each per group) and at day 7 (one mouse per group) biopsies were taken, which included the entire wound so as to obtain a homogenate of the wound and thus demonstrate the EGF protein. On the other hand, immuno-histochemical tests were carried out by means of conventional histological methods. The results are illustrated in Fig. 2. As can be seen, 24 h after transformation with the EGF-plasmid, the non-cultured human keratinocytes exhibit a 181-fold increased EGF concentration as compared to wounds transfected with the pCMVβ-Gal as the control (Fig. 4). EGF concentrations were detected for the entire 7-day testing period. Yet within the first three days they decreased rapidly, starting from 180 µg/ml at day 1 post treatment until approximately 20 µg/ml at day 7 post treatment.

After transplanting cultured human keratinocytes which had been re-suspended with the fibrin matrix, containing the EGF-plasmid, similar concentration patterns were obtained for the EGF protein (Fig. 3), (200.5 µg/ml; day 7, 20 µg/ml).

### Example 2

Determination of the optimal keratinocyte concentration for a given buffer. EGF-plasmid, Tissucol® and cell preparations were used as described in Example 1. Only the amount of keratinocytes was varied between 100 000, 1 million, 2 millions and 5 millions. The highest expression rates of EGF were obtained in using a cell number of 2 millions/333 µl of fibrin clot, which amounts to about 6 millions cells/ml of clot (see Fig. 5).

### Example 3

Optimization of a gene activated matrix for the treatment of full thickness wounds of nude mice.
Full thickness wounds of nude mice (12 mice per group) were treated with different combinations (groups 1-4). The amount of fibrin in each case was 333 µl, the amount of plasmid 200 µg. In each case a pre-incubation of appropriate cells with the plasmid was carried out in 5.7 µl PBS for 3 hours. The amount of keratinocytes used was 2 millions. Biopsies were taken on days 1, 3, 5, 7, 9 and 12 and histologies starting with day 5.

| | |
|---|---|
| Group 1 | Fibrin and EGF-plasmid |
| Group 2 | Fibrin and keratinocytes |
| Group 3 | Fibrin, EGF-plasmid and endothelial cells (=support cells) |
| Group 4 | Fibrin, EGF-plasmid and keratinocytes (=repair cells) |

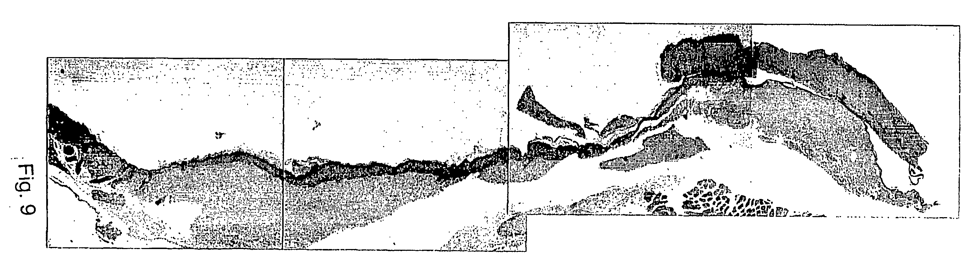

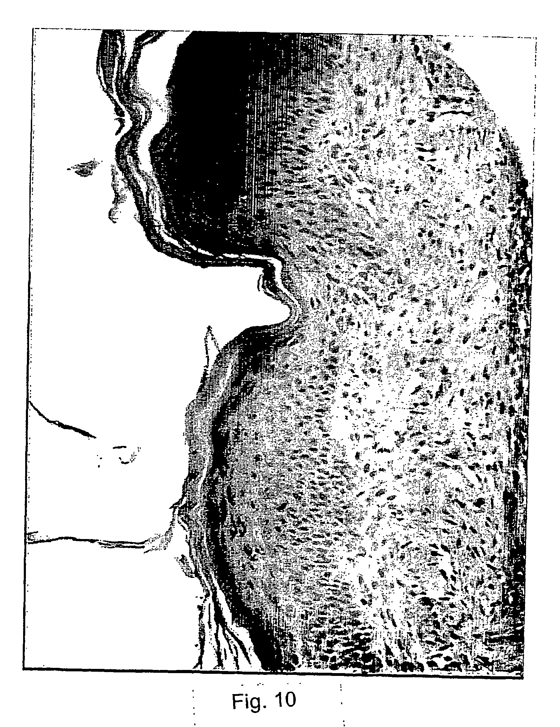

The results of histologies clearly show that only group 4 leads to a full re-epithelialization of a full thickness wounds in nude mice, having a completely regenerated epithelium consisting of 9-11 layers of cells (see Figures 9 and 10).

With all other groups (1-3) only some re-epithelialization from the edge of the wounds is visible, whereas no re-epithelialization occurred in the center of the wounds (see Figures 6, 7 and 8).

### Example 4

### Transfection of various cell types

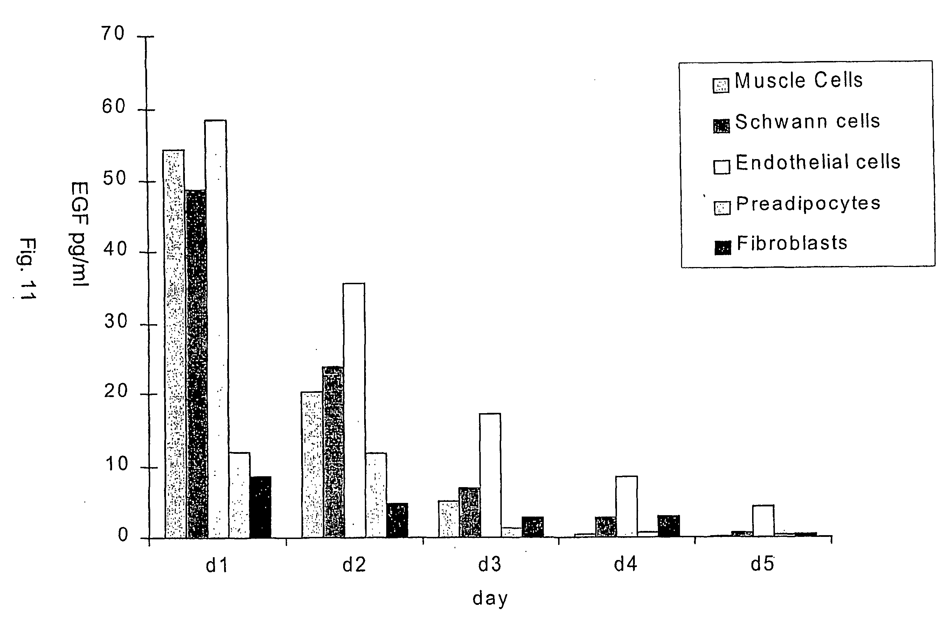

200 000 cells, namely muscle cells, Schwann cells, endothelial cells, preadipocytes and fibroblasts, where transfected with 10 µg of EGF-plasmid each, the amount of fibrin used was 333 µl. Expression of EGF was measured after day 1, 2, 3, 4 and 5 and is shown in Figure 11.

### Examples for isolation of cells

### Schwann cells

Cells were prepared with modification according to the method of Shahar et al., (1989) in which Schwann cells were harvested from the sciatic nerve of neonatal rats. In brief, the Schwann cells were harvested from 7 mm segments of the sciatic nerve. Nerves were collected in HBSS, stripped of their epineurium and chopped into 1 mm² pieces. The nerve pieces were dissociated by incubating the chunks for 30 minutes at 37°C with 0.3% trypsin and 0.1% collagenase. The cells were then triturated, washed and cultured with DMDM containing 10% FCS and penicillin/streptomycin on poly-D-lysine coated flasks. The following day, ara-c was added to the culture medium for 48 hours before the medium was replaced with mitogenic medium containing forskolin. Every 3 days the medium was changed until the Schwann cells reaches confluence.

### Human microvascular endothelial cells

After removal of fat and epidermis the dermis was chopped into approximately 4 cm² sized pieces. The endothelial cells were obtained from the dermis following the separation of dermis and epidermis after overnight incubation in dispase (2,4 U/ml) at 4°C. The dermal pieces were recut and incubated with trypsin at 37°C for 30 minutes. Pressure was applied to the dermal pieces using a scalpel to obtain the endothelial cells. The petridish was then rinsed with EGM (5%). The suspension was filtered /70 µm). After centrifugation at 1300 U/min for 5 minutes the pellet was resuspended in 5% EGM and plated on gelatin coated flasks.

## Claims

1. A method of preparing a composition for wound healing, and for repairing and regenerating mammalian tissue, said method comprising the following steps:
a. providing a plasmid DDIA in substantially pure form which encodes a gene that has a positive effect on the progression of the regeneration of the tissue,
b. providing a component/components of a self-hardening biopolymer, and
c. providing a cell suspension with cells which promote regeneration,
**characterized in that** components (a), (b) and (c) are incubated with each other simultaneously or successively so that the plasmid and the cell suspension are obtained homogenously distributed in one of the biopolymer components.

2. A method according to claim 1, **characterized in that** the biopolymer is selected from a fibrin adhesive, collagen, gelatin, alginate, hyaluronic acid, polysaccharide, organic polymer or derivatives thereof or combinations thereof, respectively.

3. A method according to claim 1 or 2, **characterized in that** the biopolymer is a fibrin adhesive.

4. A method according to any one of claims 1 to 3, **characterized in that** the plasmid and the cells are in the thrombin component of the fibrin adhesive.

5. A method according to any one of claims 1 to 3, **characterized in that** the biopolymer is provided in a liquid or lyophilized form.

6. A method according to any one of claims 1 to 5, **characterized in that** the ratio of plasmid to biopolymer component is 5-25 µg/ml, preferably 10-20 µg/ml.

7. A method according to any one of claims 1 to 6, **characterized in that** the ratio of plasmid to biopolymer component is 25-100 µg/ml, preferably around 50 µg/ml.

8. A method according to any one of claims 1 to 7, **characterized in that** the ratio is from 25,000-75,000 cells/µg of plasmid.

9. A method according to any one of claims 1 to 7, **characterized in that** the ratio is 75,000-100,000 cells/µg of plasmid, preferably around 50,000.

10. A method according to any one of the preceding claims, **characterized in that** the number of cells per ml of biopolymer component is 200,000 to 5 000,000, preferably 3 000,000.

11. A method according to any one of the preceding claims, **characterized in that** the cell suspension is a suspension of cells selected from keratinocytes, chondrocytes, fibroblasts, epithelial cells, endothelial cells, Schwann cells, osteoblasts and osteoclasts.

12. Transfection system containing a plasmid DNA in substantially pure form which codes for a gene that has a positive effect on the progression of regeneration of the tissue, a component of a self-hardening biopolymer and a cell suspension with cells promoting the regeneration and which does not contain any further transfection-promoting or transfection-mediating substances.

13. A pharmaceutical composition containing a plasmid DNA in substantially pure form which codes for a gene that has a positive effect on the progression of the regeneration of tissue, components of a self-hardening biopolymer, a cell suspension with cells which promote the regeneration and, optionally, a further pharmaceutically acceptable carrier, and no further transfection-promoting or transfection-mediating substances.

14. A therapeutical kit for treating tissue defects, comprising:
- a plasmid DNA in substantially pure form, which encodes a gene that has a positive effect on the progression of the regeneration of the tissue,
- components of a self-hardening biopolymer, and
- a cell suspension comprising cells which promote said regeneration, and which does not contain any further transfection-promoting or transfection-mediating substances.

15. A therapeutical kit containing a composition obtainable according to a method according to any one of claims 1 to 11.

16. The use of a transfection system, of the pharmaceutical composition or of the therapeutical kit according to any one of claims 12 to 15 in the manufacture of a medicament for the treatment of tissue defects.

17. The use according to claim 16, wherein the tissue defects are selected from the group consisting of burn wounds, bone, muscle, nerve or cartilage defects, chronic wounds and tissue augmentations.

18. The use according to claim 16, wherein the treatment of tissues defects is wound healing in the skin.

19. The use according to any one of claims 16 to 18, wherein the composition is to be sprayed onto the tissue defect.

20. The use of a gel containing the pharmaceutical composition obtainable according to a method according to any one of claims 1 to 11 in the manufacture of a medicament for the treatment of tissue defects.

21. The use of a composition prepared according to any one of claims 1-11, the system according to claim 12, the pharmaceutical composition according to claim 13 or the kit according to claim 14 or 15 in the manufacture of a medicament for the treatment of tissue defects.

22. The use according to claim 21, wherein the tissue defects are selected from the group consisting of burn wounds, bone, muscle, nerve or cartilage defects, chronic wounds and tissue augmentations.

23. The use according to claim 21 or 22, wherein the treatment of tissue defects is wound healing in the skin.

## Patentansprüche

1. Verfahren zum Herstellen einer Zusammensetzung zur Wundheilung und zum Reparieren und Regenerieren von Säugergewebe, wobei das Verfahren die folgenden Schritte umfaßt:
a. Bereitstellen einer Plasmid-DNA in im wesentlichen reiner Form, die ein Gen kodiert, das einen positiven Effekt auf den Verlauf der Regeneration des Gewebes aufweist,
b. Bereitstellen von einer Komponente/Komponenten eines selbsthärtenden Biopolymers und
c. Bereitstellen einer Zellsuspension mit Zellen, welche die Regeneration unterstützen,
**dadurch gekennzeichnet, daß** die Komponenten (a), (b) und (c) miteinander gleichzeitig oder nacheinander inkubiert werden, so daß das Plasmid und die Zellsuspension homogen verteilt in einer der Biopolymerkomponenten erhalten werden.

2. Verfahren nach Anspruch 1, **dadurch gekennzeichnet, daß** das Biopolymer aus einem Fibrinkleber, Kollagen, Gelatine, Alginat, Hyaluronsäure, Polysaccharid, organischem Polymer oder Derivaten davon bzw. Kombinationen davon ausgewählt ist.

3. Verfahren nach Anspruch 1 oder 2, **dadurch gekennzeichnet, daß** das Biopolymer ein Fibrinkleber ist.

4. Verfahren nach einem der Ansprüche 1 bis 3, **dadurch gekennzeichnet, daß** das Plasmid und die Zellen in der Thrombinkomponente des Fibrinklebers sind.

5. Verfahren nach einem der Ansprüche 1 bis 3, **dadurch gekennzeichnet, daß** das Biopolymer in einer flüssigen oder lyophilisierten Form bereitgestellt wird.

6. Verfahren nach einem der Ansprüche 1 bis 5, **dadurch gekennzeichnet, daß** das Verhältnis von Plasmid zu Biopolymerkomponente 5-25 µg/ml, vorzugsweise 10-20 µg/ml, ist.

7. Verfahren nach einem der Ansprüche 1 bis 6, **dadurch gekennzeichnet, daß** das Verhältnis von Plasmid zu Biopolymerkomponente 25-100 µg/ml, vorzugsweise etwa 50 µg/ml, ist.

8. Verfahren nach einem der Ansprüche 1 bis 7, **dadurch gekennzeichnet, daß** das Verhältnis von 25000 bis 75000 Zellen/µg Plasmid ist.

9. Verfahren nach einem der Ansprüche 1 bis 7, **dadurch gekennzeichnet, daß** das Verhältnis 75000 bis 100000 Zellen/µg Plasmid, vorzugsweise etwa 50000, ist.

10. Verfahren nach einem der vorhergehenden Ansprüche, **dadurch gekennzeichnet, daß** die Anzahl von Zellen pro ml Biopolymerkomponente 200000 bis 5000000, vorzugsweise 3000000, ist.

11. Verfahren nach einem der vorhergehenden Ansprüche, **dadurch gekennzeichnet, daß** die Zellsuspension eine Suspension von Zellen, ausgewählt aus Keratinozyten, Chondrozyten, Fibroblasten, epithelialen Zellen, endothelialen Zellen, Schwann-Zellen, Osteoblasten und Osteoklasten, ist.

12. Transfektionssystem, enthaltend eine Plasmid-DNA in im wesentlichen reiner Form, die ein Gen kodiert, welches einen positiven Effekt auf den Verlauf der Regeneration des Gewebes aufweist, eine Komponente eines selbsthärtenden Biopolymers und eine Zellsuspension mit Zellen, welche die Regeneration unterstützen, und welches keine weiteren Transfektion-fördernden oder Transfektion-vermittelnden Substanzen enthält.

13. Pharmazeutische Zusammensetzung, enthaltend eine Plasmid-DNA in im wesentlichen reiner Form, die ein Gen kodiert, das einen positiven Effekt auf den Verlauf der Regeneration des Gewebes aufweist, Komponenten eines selbsthärtenden Biopolymers, eine Zellsuspension mit Zellen, welche die Regeneration fördern, und gegebenenfalls einen weiteren pharmazeutisch verträglichen Träger und keine weiteren Transfektion-fördernden oder Transfektion-vermittelnden Substanzen.

14. Therapeutischer Kit zum Behandeln von Gewebedefekten, der
- eine Plasmid-DNA in im wesentlichen reiner Form, die ein Gen kodiert, das einen positiven Effekt auf den Verlauf der Regeneration des Gewebes aufweist,
- Komponenten eines selbsthärtenden Biopolymers und
- eine Zellsuspension, umfassend Zellen, welche die Regeneration unterstützen, umfaßt und der keine weiteren Transfektion-fördernden oder Transfektion-vermittelnden Substanzen enthält.

15. Therapeutischer Kit, enthaltend eine nach einem Verfahren nach einem der Ansprüche 1 bis 11 erhältliche Zusammensetzung.

16. Verwendung eines Transfektionssystems der pharmazeutischen Zusammensetzung oder des therapeutischen Kits nach einem der Ansprüche 12 bis 15 in der Herstellung eines Medikaments zum Behandeln von Gewebedefekten.

17. Verwendung nach Anspruch 16, wobei die Gewebedefekte ausgewählt sind aus der Gruppe, bestehend aus Verbrennungswunden, Knochen-, Muskel-, Nerven- oder Knorpelschäden, chronischen Wunden und Gewebeaugmentationen.

18. Verwendung nach Anspruch 16, wobei das Behandeln der Gewebedefekte eine Wundheilung in der Haut ist.

19. Verwendung nach einem der Ansprüche 16 bis 18, wobei die Zusammensetzung auf den Gewebedefekt zu sprühen ist.

20. Verwendung eines Gels, welches die nach einem Verfahren nach einem der Ansprüche 1 bis 11 erhältliche pharmazeutische Zusammensetzung enthält, in der Herstellung eines Medikaments zum Behandeln von Gewebedefekten.

21. Verwendung einer Zusammensetzung, hergestellt nach einem der Ansprüche 1 bis 11, eines Systems nach Anspruch 12, einer pharmazeutischen Zusammensetzung nach Anspruch 13 oder eines Kits nach Anspruch 14 oder 15 in der Herstellung eines Medikaments zum Behandeln von Gewebedefekten.

22. Verwendung nach Anspruch 21, wobei die Gewebedefekte ausgewählt sind aus der Gruppe, bestehend aus Verbrennungswunden, Knochen-, Muskel-, Nerven- oder Knorpelschäden, chronischen Wunden und Gewebeaugmentationen.

23. Verwendung nach Anspruch 21 oder 22, wobei das Behandeln der Gewebedefekte eine Wundheilung in der Haut ist.

## Revendications

1. Procédé pour préparer une composition de cicatrisation, et pour réparer et régénérer un tissu de mammifère, ledit procédé comprenant les étapes suivantes :
a. fourniture d'un ADN plasmidique sous forme pratiquement pure qui code un gène qui a un effet positif sur la progression de la régénération du tissu,
b. fourniture d'un ou plusieurs composants d'un biopolymère auto-durcissable, et
c. fourniture d'une suspension cellulaire avec des cellules qui favorisent la régénération,
**caractérisé en ce que** les composants (a), (b) et (c) sont incubés les uns avec les autres simultanément ou successivement de façon que le plasmide et la suspension cellulaire soient obtenus en étant distribués de façon homogène dans l'un des composants de biopolymère.

2. Procédé selon la revendication 1, **caractérisé en ce que** le biopolymère est choisi parmi un adhésif de type fibrine, le collagène, la gélatine, un alginate, l'acide hyaluronique, un polysaccharide, un polymère organique ou leurs dérivés ou leurs combinaisons, respectivement.

3. Procédé selon la revendication 1 ou 2, **caractérisé en ce que** le biopolymère est un adhésif de type fibrine.

4. Procédé selon l'une quelconque des revendications 1 à 3, **caractérisé en ce que** le plasmide et les cellules sont dans le composant thrombine de l'adhésif de type fibrine.

5. Procédé selon l'une quelconque des revendications 1 à 3, **caractérisé en ce que** le biopolymère se présente sous une forme liquide ou lyophilisée.

6. Procédé selon l'une quelconque des revendications 1 à 5, **caractérisé en ce que** le rapport du plasmide au composant de biopolymère est de 5 à 25 µg/ml, de préférence de 10 à 20 µg/ml.

7. Procédé selon l'une quelconque des revendications 1 à 6, **caractérisé en ce que** le rapport du plasmide au composant de biopolymère est de 25 à 100 µg/ml, de préférence de l'ordre de 50 µg/ml.

8. Procédé selon l'une quelconque des revendications 1 à 7, **caractérisé en ce que** le rapport est de 25 000 à 75 000 cellules/µg de plasmide.

9. Procédé selon l'une quelconque des revendications 1 à 7, **caractérisé en ce que** le rapport est de 75 000 à 100 000 cellules/µg de plasmide, de préférence de l'ordre de 50 000.

10. Procédé selon l'une quelconque des revendications précédentes, **caractérisé en ce que** le nombre de cellules par ml de composant de biopolymère est de 200 000 à 5 000 000, de préférence de 3 000 000.

11. Procédé selon l'une quelconque des revendications précédentes, **caractérisé en ce que** la suspension cellulaire est une suspension de cellules choisies parmi les kératinocytes, les chondrocytes, les fibroblastes, les cellules épithéliales, les cellules endothéliales, les cellules de Schwann, les ostéoblastes et les ostéoclastes.

12. Système de transfection contenant un ADN plasmidique sous forme pratiquement pure qui code un gène qui a un effet positif sur la progression de la régénération du tissu, un composant d'un biopolymère auto-durcissable et une suspension cellulaire avec des cellules favorisant la régénération et qui ne contient aucune autre substance favorisant la transfection ou ayant un effet médiateur sur la transfection.

13. Composition pharmaceutique contenant un ADN plasmidique sous forme pratiquement pure qui code un gène qui a un effet positif sur la progression de la régénération du tissu, des composants d'un biopolymère auto-durcissable, une suspension cellulaire avec des cellules qui favorisent la régénération et, éventuellement, un autre véhicule pharmaceutiquement acceptable, et aucune autre substance favorisant la transfection ou ayant un effet médiateur sur la transfection.

14. Kit thérapeutique pour traiter des défauts tissulaires, comprenant :
- un ADN plasmidique sous forme pratiquement pure, qui code un gène qui a un effet positif sur la progression de la régénération du tissu,
- des composants d'un biopolymère auto-durcissable, et
- une suspension cellulaire comprenant des cellules qui favorisent ladite régénération, et qui ne contient aucune autre substance favorisant la transfection ou ayant un effet médiateur sur la transfection.

15. Kit thérapeutique contenant une composition pouvant être obtenue conformément à un procédé selon l'une quelconque des revendications 1 à 11.

16. Utilisation d'un système de transfection, de la composition pharmaceutique ou du kit thérapeutique selon l'une quelconque des revendications 12 à 15, dans la fabrication d'un médicament destiné au traitement de défauts tissulaires.

17. Utilisation selon la revendication 16, dans laquelle les défauts tissulaires sont choisis dans le groupe constitué par les brûlures, les défauts osseux, musculaires, nerveux ou cartilagineux, les plaies chroniques et les augmentations de tissu.

18. Utilisation selon la revendication 16, dans laquelle le traitement de défauts tissulaires est une cicatrisation dans la peau.

19. Utilisation selon l'une quelconque des revendications 16 à 18, dans laquelle la composition est à pulvériser sur le défaut tissulaire.

20. Utilisation d'un gel contenant la composition pharmaceutique pouvant être obtenue conformément à un procédé selon l'une quelconque des revendications 1 à 11, dans la fabrication d'un médicament destiné au traitement de défauts tissulaires.

21. Utilisation d'une composition préparée conformément à l'une quelconque des revendications 1 à 11, du système selon la revendication 12, de la composition pharmaceutique selon la revendication 13 ou du kit selon la revendication 14 ou 15, dans la fabrication d'un médicament destiné au traitement de défauts tissulaires.

22. Utilisation selon la revendication 21, dans laquelle les défauts tissulaires sont choisis dans le groupe constitué par les brûlures, les défauts osseux, musculaires, nerveux ou cartilagineux, les plaies chroniques et les augmentations de tissu.

23. Utilisation selon la revendication 21 ou 22, dans laquelle le traitement de défauts tissulaires est une cicatrisation dans la peau.
